Europäisches Patentamt

European Patent Office

Office européen des brevets

(11) Veröffentlichungsnummer : **0 053 713**
**B1**

(12) ## EUROPÄISCHE PATENTSCHRIFT

(45) Veröffentlichungstag der Patentschrift :
04.07.84

(51) Int. Cl.³ : **C 07 C103/38**, C 07 C102/00

(21) Anmeldenummer : **81109188.3**

(22) Anmeldetag : **29.10.81**

(54) **Verfahren zur Herstellung von 1-Acetoacetylamino-4-chlor-2,5-dimethoxybenzol.**

(30) Priorität : **09.12.80 DE 3046367**

(43) Veröffentlichungstag der Anmeldung :
**16.06.82 Patentblatt 82/24**

(45) Bekanntmachung des Hinweises auf die Patenterteilung : **04.07.84 Patentblatt 84/27**

(84) Benannte Vertragsstaaten :
**CH DE FR GB LI**

(56) Entgegenhaltungen :
**EP-A- 0 001 994**
**DE-A- 2 518 984**
**DE-A- 2 519 036**

(73) Patentinhaber : **HOECHST AKTIENGESELLSCHAFT**
**Postfach 80 03 20**
**D-6230 Frankfurt am Main 80 (DE)**

(72) Erfinder : **Heise, Harmut, Dr.**
**Am Rehsteig 8**
**D-6232 Bad Soden am Taunus (DE)**
Erfinder : **Mörler, Dieter, Dr.**
**In den Eichen 39**
**D-6237 Liederbach (DE)**

# 0 053 713

**Beschreibung**

1-Acetoacetylamino-4-chlor-2,5-dimethoxybenzol ist eine häufig verwendete Kupplungskomponente (C.I. Azoic Coupling Component 44, C.I. 37613) für Azofarbmittel, insbesondere für Azopigmente.

Aus der DE-PS 25 18 984 ist es bekannt, 1-Acetoacetylamino-4-chlor-2,5-dimethoxybenzol durch Suspendieren von 2,5-Dimethoxy-4-chloranilin in Wasser und Umsetzen mit Diketen herzustellen, wobei das Diketen gleich zu Beginn der Reaktion auf einmal zugegeben wird. Die Umsetzung wird durch Säure katalysiert, wobei organische Säuren bevorzugt werden. Auf 1 Mol Amin werden zweckmäßig 0,1 bis 1 Mol Säure eingesetzt. In den Beispielen arbeitet man mit Essigsäurekonzentrationen bis etwa 5 %.

Bei diesem Verfahren wird das Produkt zwar in hoher Ausbeute erhalten, aber die Reinheit des Produkts genügt nicht den Ansprüchen, die an ein Ausgangsmaterial zur Synthese hochechter Azopigmente gestellt werden.

Aus der Patentanmeldung EP-A1 0 001 994 ist bekannt, 1-Acetoacetylamino-2,5-dimethoxy-4-chlorbenzol durch Umsetzung von 2,5-Dimethoxy-4-chloranilin mit Diketen in inerten organischen Lösungsmitteln, wie aliphatische Kohlenwasserstoffe, aromatische Kohlenwasserstoffe oder inerte Chlorkohlenwasserstoffe, in Gegenwart katalytischer Mengen Wasser und einer Carbonsäure herzustellen.

Hierbei wird das Produkt zwar in guter Reinheit erhalten, jedoch muß zur Erzielung einer hohen Ausbeute und für die Wiederverwendung das gesamte Lösungsmittel eines jeden Ansatzes aufgearbeitet werden. Außerdem fällt das Produkt lösungsmittelfeucht an und muß vor der Weiterverarbeitung in jedem Fall getrocknet werden.

Es wurde nun gefunden, daß man in einfacher Weise unter Vermeidung der Nachteile der vorstehend genannten, zum Stand der Technik zählenden Verfahren 1-Acetoacetylamino-4-chlor-2,5-dimethoxybenzol in hoher Qualität aus 1-Amino-4-chlor-2,5-dimethoxybenzol und Diketen in wäßriger Essigsäure erhält, wenn die Umsetzung bei 20 bis 90 °C in Essigsäure einer Konzentration von 35 bis 70 Gew.-%, die zu 60 bis 90 Gew.-% aus der Mutterlauge eines vorhergehenden Ansatzes besteht, erfolgt, worauf man das Produkt bei 0 bis 25 °C kristallisieren läßt und 10 bis 40 Gew.-% der anfallenden Mutterlauge ausschleust. Die ausgeschleuste Mutterlauge wird zweckmäßig regeneriert, beispielsweise durch Destillation, wobei die so gewonnene Essigsäure wieder anteilig als Reaktionsmedium verwendet werden kann.

Es hat sich gezeigt, daß die erfindungsgemäße Arbeitsweise Ausbeuten in der Größenordnung von 90 % und Produktqualitäten ergibt, die ohne Reinigung den Anforderungen entsprechen, die an Kupplungskomponenten für hochechte Azopigmente gestellt werden.

Im folgenden werden bevorzugte Ausgestaltungen der Erfindung näher erläutert :

Die Umsetzungstemperatur, die Essigsäurekonzentration und der Anteil der Mutterlauge am Reaktionsmedium sowie die Konzentration der Reaktionsteilnehmer werden zweckmäßig so aufeinander abgestimmt, daß am Ende der Umsetzung das Produkt praktisch vollständig gelöst ist. Es empfiehlt sich, ungelöste Bestandteile abzutrennen, was bevorzugt in Form einer Klärfiltration erfolgt. Bevorzugt werden hierbei Klärhilfsmittel wie Kieselgur, insbesondere Aktivkohle, zugesetzt.

Aus der geklärten Lösung läßt man dann das Produkt durch Abkühlung, bevorzugt auf einen Temperaturbereich von 5 bis 15 °C, kristallisieren.

Das auskristallisierte Produkt wird von der Mutterlauge abgetrennt, von der 10 bis 40 Gew.-% ausgeschleust werden. Der Rest wird zweckmäßig einem folgenden Ansatz zugeführt. Durch das Ausschleusen eines Teils der Mutterlauge wird eine Anreicherung von Verunreinigungen vermieden und eine gleichbleibende Qualität des Produkts gewährleistet.

Zweckmäßig wird das isolierte Produkt mit Essigsäure der gleichen Konzentration wie das Reaktionsmedium gewaschen. Die Waschessigsäure wird zweckmäßig wie die Mutterlauge weiterbehandelt, das heißt in den Prozeß zurückgeführt und/oder regeneriert.

Sofern das Produkt nicht unmittelbar weiterverarbeitet wird, kann es durch Waschen mit Wasser von der anhaftenden Essigsäure befreit und entweder in dieser Form weiterverarbeitet oder getrocknet werden.

Wie vorstehend erwähnt, kann die Konzentration der Essigsäure und die Reaktionstemperatur in gewissen Grenzen schwanken. Bevorzugt wird eine Essigsäurekonzentration von 45 bis 60 Gew.-% und eine Reaktionstemperatur von 25 bis 70 °C. Abhängig von diesen Parametern wird die Konzentration des Amins in der Weise gewählt, daß das Produkt am Ende der Umsetzung gelöst ist. Andererseits soll bei der Kristallisation das Produkt in hoher Reinheit und guter Ausbeute anfallen. Die Essigsäure spielt hierbei die Rolle eines Lösevermittlers ; sie wird in einer Menge von mindestens 5, vorzugsweise von etwa 7,5 bis etwa 13 Mol pro Mol Amin eingesetzt.

Das Diketen wird zweckmäßig in einem geringen Überschuß von vorzugsweise etwa 5 bis 10, insbesondere etwa 6 bis 8 Gew.-% verwendet.

Als Reaktions- und Kühlgefäße eignen sich übliche Rührbehälter aus nicht korrodierenden Werkstoffen wie Email oder Edelstahl. Das Verfahren kann kontinuierlich oder diskontinuierlich ausgestaltet werden.

In den folgenden Beispielen beziehen sich Prozentangaben auf das Gewicht, sofern nichts anderes angegeben ist. Eingesetzt wurde technisch reines Diketen (etwa 98 %ig).

2

## Beispiel 1

In einem 8,5 m³ fassenden Edelstahl-Rührbehälter werden 1 200 l (frische oder regenerierte) 50 %ige Essigsäure und 3 600 l Mutterlauge aus einem vorhergehenden Ansatz (entsprechend 50 %iger Essigsäure) vorgelegt und 700 kg 1-Amino-4-chlor-2,5-dimethoxybenzol eingetragen. Die Mischung wird auf 25 °C erwärmt. Dann läßt man innerhalb von 30 Minuten 345 kg Diketen zulaufen und den Ansatz 15 Minuten nachreagieren. Dann werden 20 kg Aktivkohle zugegeben, die Mischung auf 50 °C geheizt und über ein Filter in einen zweiten 8,5 m³ fassenden Edelstahl-Rührbehälter geklärt. Nach Prüfung auf vollständigen Umsatz wird die Mischung auf 10 °C abgekühlt und das auskristallisierte Reaktionsprodukt abfiltriert. Der Filterrückstand wird mit 500 l 50 %iger Essigsäure gewaschen und das Filtrat mit der Mutterlauge vereinigt. Hiervon werden 3 600 l dem nächsten Ansatz zugeführt und der Rest in eine Destillationskolonne geleitet, wo er zu 50 %iger Essigsäure aufgearbeitet wird, die wieder in den Prozeß zurückgeführt bzw. zum Waschen des Produkts verwendet werden kann. Der Filterrückstand wird hierauf mit wenig Wasser essigsäurefrei gewaschen. Man erhält 1 140 kg 1-Acetoacetylamino-4-chlor-2,5-dimethoxybenzol als 80 %ige Feuchtware entsprechend 910 kg Reinprodukt (89,8 % d. Th.), das in dieser Form oder nach Trocknung ohne weitere Reinigung zur Herstellung hochechter Azopigmente verwendbar ist.

Die folgenden Beispiele wurden gemäß Beispiel 1 durchgeführt :

(Siehe Tabelle Seite 4 f.)

| Bsp. | Konz. Essigs. (%) | Menge an Essigs. (l) Mutterl. | Regen. | Amin (kg) | Diketen (kg) | Temp. beim Klären (°C) | Ausbeute kg | % d. Th. |
|---|---|---|---|---|---|---|---|---|
| 2 | 40 | 4 300 | 500 | 700 | 345 | 55 | 915 | 90,3 |
| 3 | 50 | 3 800 | 1 000 | 800 | 395 | 50 | 1 048 | 90,5 |
| 4 | 60 | 3 800 | 1 000 | 800 | 395 | 50 | 1 017 | 87,8 |
| 5 | 70 | 4 300 | 500 | 900 | 445 | 50 | 1 117 | 85,7 |
| 6 | 50 | 3 600 | 1 200 | 900 | 445 | 60 | 1 194 | 91,6 |
| 7 | 50 | 3 600 | 1 200 | 1 000 | 493 | 70 | 1 332 | 92,0 |
| 8 | 50 | 3 300 | 1 500 | 1 000 | 493 | 70 | 1 306 | 90,2 |
| 9 | 50 | 2 900 | 1 900 | 1 000 | 493 | 70 | 1 265 | 87,4 |

0 053 713

## Ansprüche

1. Verfahren zur Herstellung von 1-Acetoacetylamino-4-chlor-2,5-dimethoxybenzol aus 1-Amino-4-chlor-2,5-dimethoxybenzol und Diketen in wäßriger Essigsäure, dadurch gekennzeichnet, daß die Umsetzung bei 20 bis 90 °C in Essigsäure einer Konzentration von 35 bis 70 Gew.-%, die zu 60 bis 90 Gew.-% aus der Mutterlauge eines vorhergehenden Ansatzes besteht, erfolgt, worauf man das Produkt bei 0 bis 25 °C kristallisieren läßt und 10 bis 40 Gew.-% der anfallenden Mutterlauge ausschleust.

2. Verfahren nach Anspruch 1, dadurch gekennzeichnet, daß die Umsetzung bei 25 bis 70 °C erfolgt.

3. Verfahren nach Anspruch 1 und 2, dadurch gekennzeichnet, daß die Umsetzung in Essigsäure einer Konzentration von 45 bis 60 Gew.-% erfolgt.

4. Verfahren nach Anspruch 1 bis 3, dadurch gekennzeichnet, daß man das Produkt bei 5 bis 15 °C kristallisieren läßt.

5. Verfahren nach Anspruch 1 bis 4, dadurch gekennzeichnet, daß die Produktlösung vor der Kristallisation einer Klärfiltration unterworfen wird.

6. Verfahren nach Anspruch 5, dadurch gekennzeichnet, daß ein Klärhilfsmittel zugesetzt wird.

7. Verfahren nach Anspruch 5 und 6, dadurch gekennzeichnet, daß als Klärhilfsmittel Aktivkohle zugesetzt wird.

8. Verfahren nach Anspruch 1 bis 7, dadurch gekennzeichnet, daß das isolierte Produkt mit wäßriger Essigsäure gewaschen wird.

9. Verfahren nach Anspruch 8, dadurch gekennzeichnet, daß die Essigsäure die gleiche Konzentration wie das Reaktionsmedium hat.

10. Verfahren nach Anspruch 8 und 9, dadurch gekennzeichnet, daß das Reaktionsprodukt mit Wasser neutral gewaschen wird.

## Claims

1. A process for the preparation of 1-acetoacetylamino-4-chloro-2,5-dimethoxybenzene from 1-amino-4-chloro-2,5-dimethoxybenzene and diketene in aqueous acetic acid, which comprises carrying out the reaction at 20 to 90 °C in acetic acid of a concentration of 35 to 70 % by weight consisting of 60 to 90 % by weight of the mother liquor of a previous batch, after which the product is crystallized at 0 to 25 °C and 10 to 40 % by weight of the mother liquor produced is discharged.

2. A process as claimed in claim 1, wherein the reaction is carried out at 25 to 70 °C.

3. A process as claimed in claim 1 and 2, wherein the reaction is carried out in acetic acid of a concentration of 45 to 60 % by weight.

4. A process as claimed in any of claims 1 to 3, wherein the product is allowed to crystallize at 5 to 15 °C.

5. A process as claimed in any of claims 1 to 4, wherein the solution of product is subjected to clarification by filtration before crystallization.

6. A process as claimed in claim 5, wherein a clarifying auxiliary is added.

7. A process as claimed in claim 5 and 6, wherein active charcoal is added as the clarifying auxiliary.

8. A process as claimed in any of claims 1 to 7, wherein the product which has been isolated is washed with aqueous acetic acid.

9. A process as claimed in claim 8, wherein the acetic acid has the same concentration as the reaction medium.

10. A process as claimed in claim 8 and 9, wherein the reaction product is washed with water until it is neutral.

## Revendications

1. Procédé de préparation du 1-acétoacétylamino-4-chloro-2,5-diméthoxybenzène à partir du 1-amino-4-chloro-2,5-diméthoxybenzène et de dicétène dans de l'acide acétique aqueux, procédé caractérisé en ce que l'on effectue la réaction entre 20 et 90 °C dans de l'acide acétique à une concentration de 35 à 70 % en poids, acide constitué pour 60 à 90 % en poids par la liqueur mère provenant d'une opération précédente, puis on laisse cristalliser le produit entre 0 et 25 °C et on élimine 10 à 40 % en poids de la liqueur mère.

2. Procédé selon la revendication 1, caractérisé en ce que la réaction est effectuée entre 25 et 70 °C.

3. Procédé selon la revendication 1 ou 2, caractérisé en ce que la réaction est effectuée dans de l'acide acétique à une concentration de 45 à 60 % en poids.

4. Procédé selon l'une quelconque des revendications 1 à 3, caractérisé en ce qu'on laisse cristalliser le produit entre 5 et 15 °C.

5. Procédé selon l'une quelconque des revendications 1 à 4, caractérisé en ce qu'avant la cristallisation, on soumet la solution du produit à une filtration clarifiante.

6. Procédé selon la revendication 5, caractérisé en ce que l'on ajoute un agent de clarification.

7. Procédé selon la revendication 5 ou 6, caractérisé en ce que l'on ajoute du charbon actif comme agent clarifiant.

8. Procédé selon l'une quelconque des revendications 1 à 7, caractérisé en ce qu'on lave le produit isolé avec de l'acide acétique aqueux.

9. Procédé selon la revendication 8, caractérisé en ce que l'acide acétique de lavage a la même concentration que le milieu réactionnel.

10. Procédé selon la revendication 8 ou 9, caractérisé en ce qu'on lave ensuite le produit à l'eau jusqu'à neutralité.